(19) Europäisches Patentamt
European Patent Office
Office européen des brevets

(11) **EP 4 440 422 B1**

(12) **EUROPEAN PATENT SPECIFICATION**

(45) Date of publication and mention
of the grant of the patent:
**14.01.2026 Bulletin 2026/03**

(51) International Patent Classification (IPC):
***A61B 5/021*** (2006.01)      ***A61B 5/0215*** (2006.01)
***A61B 5/00*** (2006.01)      ***A61M 25/00*** (2006.01)

(21) Application number: **22821366.6**

(52) Cooperative Patent Classification (CPC):
**A61B 5/02116; A61B 5/0215; A61B 5/6852;
A61B 5/747;** A61B 5/02156

(22) Date of filing: **21.11.2022**

(86) International application number:
**PCT/EP2022/082526**

(87) International publication number:
**WO 2023/099246 (08.06.2023 Gazette 2023/23)**

(54) **METHOD AND SYSTEM TO DETECT ARTERIAL BLOOD PRESSURE DAMPING AND TO PERFORM AUTOMATED FAST FLUSHING OF THE CATHETER-TUBING**

VERFAHREN UND SYSTEM ZUR ERKENNUNG VON ARTERIELLER BLUTDRUCKDÄMPFUNG UND ZUR DURCHFÜHRUNG EINER AUTOMATISIERTEN SCHNELLEN SPÜLUNG DER KATHETERRÖHRE

PROCÉDÉ ET SYSTÈME PERMETTANT DE DÉTECTER L'AMORTISSEMENT DE LA PRESSION ARTÉRIELLE ET D'EFFECTUER UN RINÇAGE RAPIDE AUTOMATISÉ DE LA TUBULURE DU CATHÉTER

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **30.11.2021 US 202163284220 P**

(43) Date of publication of application:
**09.10.2024 Bulletin 2024/41**

(73) Proprietor: **Koninklijke Philips N.V.
5656 AG Eindhoven (NL)**

(72) Inventors:
 • **ZONG, Wei
 5656 AG Eindhoven (NL)**
 • **GREGG, Richard Earl
 5656 AG Eindhoven (NL)**
 • **GROSS, Brian David
 5656 AG Eindhoven (NL)**

(74) Representative: **Philips Intellectual Property &
Standards
High Tech Campus 52
5656 AG Eindhoven (NL)**

(56) References cited:
 **US-A1- 2010 094 113      US-A1- 2015 126 880**

 • **GUIGUE LISA ET AL: "Analysis and experimental
simulation of blood pressure signal distortions
observed on intensive care patients monitored
by a catheter in radial artery", 2016 38TH
ANNUAL INTERNATIONAL CONFERENCE OF
THE IEEE ENGINEERING IN MEDICINE AND
BIOLOGY SOCIETY (EMBC), IEEE, 16 August
2016 (2016-08-16), pages 2335 - 2338,
XP032979644, DOI: 10.1109/EMBC.2016.7591198**

**Description**

**FIELD**

The following relates generally to medical arts, medical monitoring arts, physiological monitoring arts, patient safety arts, and related arts. More particularly, embodiments herein relate to the use of automated flushing to maintain a consistent arterial blood pressure (ABP) signal for patient monitoring.

**BACKGROUND**

Invasive arterial (intra-arterial) blood pressure (IABP) monitoring is a commonly used technique in the intensive care unit (ICU). Such arterial blood pressure (ABP) monitoring is also often used in the operating room, when hemodynamic instability is a risk or when beat to beat measurements and visualization of the pressure waveform are helpful, for example.

In current clinical practice, ABP signals are typically measured with an arterial catheter and an external pressure sensor. For example, ABP monitoring usually involves the insertion of a catheter connected to a tubing system into a suitable artery (e.g., such as a radial artery) where the tubing system is also operatively associated with the external pressure sensor.

Such ABP signals can degrade over time. For example, ABP signals can degrade over time due to clot formation and/or other factors. Such a degradation in ABP signals potentially prevent and/or dampen the transmission of pressure from the artery to the external pressure sensor. Accordingly, such a degradation in ABP signals may cause incorrect ABP measurements and lead to false ABP alarms and/or misinterpretation of patient hemodynamic status.

As discussed above, such ABP damping is an abnormal measuring condition in invasive ABP monitoring. Such ABP damping needs to be identified and fixed in time to avoid leading to incorrect interpretation of a patient hemodynamic condition and/or causing false alarms. As such ABP damping situations are typically manually observed/identified, such ABP damping situations could be overlooked or misidentified with delays due to human error and/or fatigue.

Furthermore, flushing operations in response to ABP damping situations have typically also been done manually. Accordingly, the related clinical workflow may be negatively affected, and clinical workload may be increased.

US 2015/126880 A1 discloses a method for continuously monitoring a blood pressure measurement using a sensor connected by a hydraulic link to an arterial catheter, the method includes determining the dynamic parameters of the link, analyzing the frequency content of the incident signal, detecting one of the following situations: (a) ability of the link to transmit the incident signal with a distortion below a threshold, (b) ability of the link to transmit the incident signal with a distortion above the threshold and possibility of correcting the measured signal, (c) inability of the link to transmit the incident signal with a distortion below the threshold and impossibility of correcting the measured signal, periodic application of a mechanical action to the tubing providing the hydraulic link.

US 2010/094113 A1 discloses methods and apparatuses that can provide measurement of analytes such as glucose with a variety of sensors in connection with hemodynamic monitoring. An example apparatus comprises a blood access system, adapted to remove blood from a body and infuse at least a portion of the blood back into the body. Such an apparatus also comprises an analyte sensor, mounted with or integrated into the blood access system such that the analyte sensor measures the analyte in the blood that has been removed from the body by the blood access system.

The following discloses certain improvements to overcome these problems and others.

**SUMMARY**

As discussed above, arterial blood pressure damping identification and fast-flush operations are typically manually performed in current clinical practice. Advantageously, some embodiments described herein present a method and system for automated detection of arterial blood pressure damping and automated flushing in response.

As will be discussed in greater detail below, some embodiments described herein present a method and system to detect arterial blood pressure signal damping events and automatically flush the catheter tubing in response. Additionally, or alternatively, some implementations herein may evaluate the effectiveness of the automated flushing and send a notification when the damped arterial blood pressure signal is not resolved after the automated flushing. Furthermore, some implementations herein may evaluate the effectiveness of the automated flushing and start another iteration of automated flushing and tracking (e.g., reevaluate) the effectiveness of the additional flushing. In such an example, in the case when N-times (e.g., where N may be defined by a user, etc.) of automated flushing are not effective, a notification with alarm message will be sent.

According to the present invention , a method, a system, a machine-readable storage and an apparatus are presented as defined in the claims.

These and other aspects of the various embodiments will be apparent from and elucidated with reference to the embodiment(s) described hereinafter.

## BRIEF DESCRIPTION OF THE DRAWINGS

The various advantages of the embodiments will become apparent to one skilled in the art by reading the following specification and appended claims, and by referencing the following drawings, in which:

FIG. 1 is an illustration of a block diagram of an example arterial blood pressure monitor;
FIG. 2 is an illustration of a graph of an arterial blood pressure signal having an arterial blood pressure damping event;
FIG. 3 is an illustration of a block diagram of an example arterial blood pressure management system according to an embodiment;
FIG. 4 is an illustration of a flowchart of an example method of operating an arterial blood pressure management system according to an embodiment;
FIG. 5 is a further illustration of a flowchart of an example method of operating an arterial blood pressure management system according to an embodiment;
FIG. 6 is an illustration of a graph of an example detection of an arterial blood pressure damping event and a consequent assessment of the recovery for the damping event after a corresponding flush according to an embodiment;
FIG. 7 is another illustration of a graph of an example detection of an arterial blood pressure damping events and a consequent assessment of the recovery of the damping event after corresponding repeated flushes according to an embodiment;
FIG. 8 is an illustration of a block diagram of a computer program product according to an embodiment;
FIG. 9 is a further illustration of a EMR management system according to an embodiment; and
FIG. 10 is an illustration of a hardware apparatus including a semiconductor package according to an embodiment.

## DETAILED DESCRIPTION

The invention is defined by the appended claims.

As will be described in greater detail below, in some implementations discussed herein, once an ABP damping situation is detected, a control signal may be issued to activate an automated fast-flushing operation. The ABP signal may be continued to be analysed right after the automated flushing and evaluated to determine whether the damped ABP signal is resolved (e.g., returned to normal). If the dampened ABP signal is resolved after the auto-flushing, the IABP monitoring process continues. If the dampened ABP signal is not resolved, the algorithm may take another auto-flushing action, and evaluate the effectiveness of the flushing again. The automated flushing and evaluation may be tried N times (e.g., where N is a selectable number, e.g., N = 3). If after N times of auto-flushing are failed, the algorithm may then issue an alarm/alert to clinician for the ABP damping. Note that the manual flushing operation may also be available in addition to the auto-flushing action, so that a clinician may still perform manual flushing if she/he thinks necessary.

In operation, some of the techniques described herein may advantageously relieve a clinician's burden to manually observe and identify ABP damping situations. Additionally, or alternatively, some of the techniques described herein may advantageously detect the ABP damping situation in a timely fashion, avoiding delays for flushing. Further, some of the techniques described herein may advantageously reduce false ABP alarms caused by ABP damping. Additionally, some of the techniques described herein may advantageously relieve a clinician's burden of manually flushing tubing, thereby improving the related clinical workflow. Lastly, if the some of the techniques described herein detect a fake ABP damping event (e.g., a false positive) and make an auto-flushing in error, there may be limited adverse consequences, because the auto-flushing may happen in a very short period of time (e.g., 2-3 seconds) and the interruption of the IABP monitoring would be very minor.

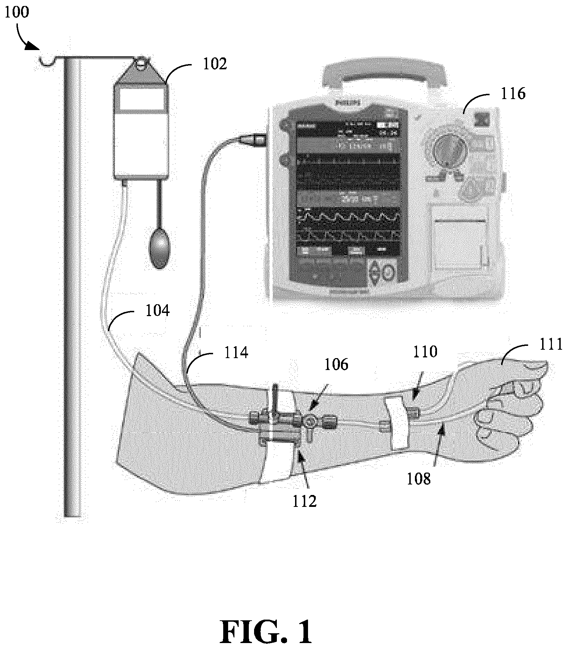

FIG. 1 shows an illustration of a block diagram of an example arterial blood pressure monitor 100. In the illustrated example, arterial blood pressure monitor 100 may include a pressure bag 102 (e.g., containing a pressurized saline solution), a flush tubing 104, stopcocks 106, catheter tubing (e.g., pressure tubing) 108, an intra-arterial cannula 110, a pressure sensor (e.g., a pressure transducer) 112, a pressure sensor receptor (e.g., a pressure cable) 114, and a pressure signal processing/display unit (monitor) 116 associated with a patient 111. The arterial blood pressure (ABP) is transmitted from the artery through a column of non-compressible, bubble free fluid (e.g., 0.9% saline) in the catheter tubing 108 to the pressure sensor 112. The flush tubing 104 is connected to the pressure bag 102 (e.g., containing a pressurized saline solution), which is usually pressurized to 300mmHg, and attached to the catheter tubing 108 via a flush system (e.g., stopcocks 106). The flush system allows a high-pressure flush of fluid in order to keep the catheter tubing 108 clear and to check the dynamic characteristics (e.g., damping and natural frequency) of the system.

In operation, with necessary initial settings of the tubing system, the invasive blood pressure (IABP) monitoring may be performed continuously for hours or even days. During such continuous monitoring, when a clot forms at a catheter tip, it can dampen the dynamic nature of the pressure transmitted to a sensor, resulting in a damped pressure signal. There may be some other factors which would cause the ABP damping problem: there could be air bubbles in the tubing, the tubing could get kinked, the catheter could have moved to a location where fluid movement is impeded, or blood could have moved into the catheter. The damped ABP signal may cause incorrect blood pressure (BP) measurements (e.g., especially the systolic and diastolic BP), leading to false ABP alarms and/or causing misinterpretation of the haemodynamic situation.

A clinician who operates IABP monitoring may need to pay attention to the ABP damping situation, usually by observing the ABP waveform. When there is an ABP damping suspected, the clinician often needs to take an action of a fast flush, which allows the pressurized saline to go through the catheter tubing for a short period of time (e.g., 2-3 second) so as to clear the tubing (e.g., which might be blocked by the clot). After a fast flush, if the damped ABP signal is resolved (e.g., returned to normal dynamic status), the IABP monitoring may continue; otherwise, another fast flush may need to be performed. In cases where multiple fast-flushes do not resolve the dampened ABP signal, a new set of complete settings of the IABP tubing system may be needed.

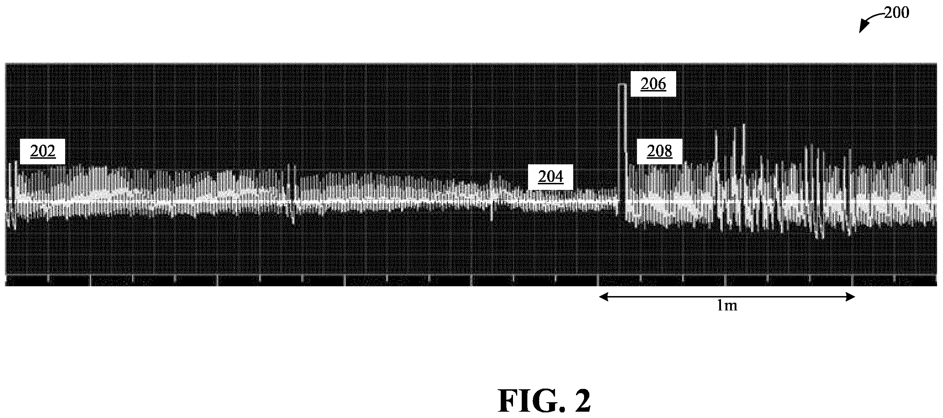

FIG. 2 shows an illustration of a graph 200 of an arterial blood pressure signal having an arterial blood pressure damping event. FIG. 2 shows an actual case in which a damped ABP episode was identified, and a fast flush was operated. After the flush, the ABP was recovered. In the illustrated example, an ABP signal 202 is illustrated as having damped ABP 204. A flush 206 is illustrated as being performed (e.g., a manual flush by a care provider) resulting in a recovered ABP 208.

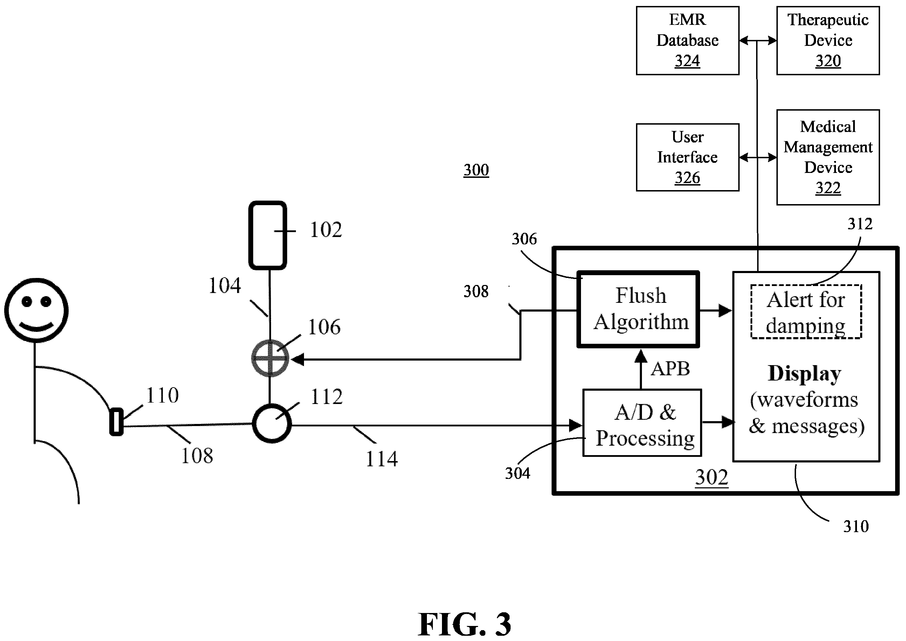

FIG. 3 shows an illustration of a block diagram of an example arterial blood pressure management system 300 according to an embodiment. In the illustrated example the arterial blood pressure management system 300 may be centralized or may be distributed and may include some or all elements and components of one or more computers or computer systems. For example, the arterial blood pressure management system 300 may include a server computer or a plurality of server computers (e.g., interconnected to form a server cluster, cloud computing resource, the like, and/or combinations thereof).

In some implementations, the arterial blood pressure management system 300 may be utilized as a control point element of an Integrated Clinical Environment (ICE). As used herein, the "Integrated Clinical Environment (ICE)" refers to a platform to create a medical Internet of Things (IoT) associated with the care of a patient. In such an implementation, the arterial blood pressure management system 300 may support many real-time clinical decision support algorithm and closed loop control algorithms of medical devices in the ICE.

In the illustrated implementation, the arterial blood pressure management system 300 may include a pressure sensor receptor 114 and an arterial blood pressure monitor 302 communicatively coupled to the pressure sensor input of the pressure sensor receptor 114. The pressure sensor receptor 114 may receive an arterial blood pressure signal detected by a pressure sensor 112.

In the illustrated implementation, the arterial blood pressure monitor 302 may include a processing module 304, a flushing algorithm 306, a display 310 (e.g., which may present an alert 312). For example, the processing module 304 may perform amplification, digitization, filtering, and scaling of the arterial blood pressure signal received from the pressure sensor 112 via the pressure sensor receptor 114; the processing module 304 may also perform pulse detection and feature extraction and may provide a scaled arterial blood pressure signal to other connected/coupled components. For example, arterial blood pressure monitor 302 may be implemented as an invasive blood pressure (IABP) monitor.

As will be discussed in greater detail below, the flushing algorithm 306 may trigger alerts and/or automated flushing in response to the arterial blood pressure signal from the processing module 304. For example, the flushing algorithm 306 may perform ABP damping detection by analying ABP waveform features in real-time. In some implementations, flushing algorithm 306 may perform detection of ABP pulses, assessment of signal quality of ABP pulses, extraction of ABP pulse features, detection of ABP damping event via recognition of the ABP damping feature patterns, the like, and/or combinations thereof. As will be described in greater detail below, an automated flushing apparatus may include a computer controlled electric fluid value in the flush tubing. Upon receiving an activation signal from the flushing algorithm 306 of an ABP damping detection, the automated flushing apparatus may make a timed (e.g., in terms of the time and duration) fast flush. Additionally, or alternatively, the flushing algorithm 306 may detect ABP recovery by tracking the ABP signal right after an automated flushing and determining whether the damped ABP signal is resolved. That the ABP signal returns to non-damping condition has distinguishable signatures (e.g., the systolic BP and pulse BP values are significantly enlarged, the maximum ABP waveform slope is significantly increased, etc.).

In the illustrated implementation, the arterial blood pressure monitor 302 may determine whether an arterial blood pressure signal damping event is occurring based on the arterial blood pressure signal. The arterial blood pressure monitor 302 may transmit an instruction to an automated valve 106 (e.g., an electronically controllable valve, such as a computer controlled electric fluid value) to automatically flush a catheter tubing 108 in response to a determination that the arterial blood pressure signal damping event is occurring. The arterial blood pressure monitor 302 may determine whether the arterial blood pressure signal damping event is resolved in response to the automatic flush of the catheter tubing 108 based on the arterial blood pressure signal. In such an example, the determination that the arterial blood pressure signal damping event is occurring may be based on a first criterion and a determination that the arterial blood pressure signal damping event is resolved is based on a second criterion that is different than the first criterion.

In operation, the arterial blood pressure monitor 302 may determine, with regard to the first criterion, whether the arterial blood pressure signal damping event is occurring based on a specific pattern change of the arterial blood pressure signal within a specified time window and beyond one or more specified threshold values. Additionally, or alternatively, the arterial blood pressure monitor 302 may determine, with regard to the second criterion, whether the arterial blood pressure signal occurring before a specified pre-flush time window compared to the arterial blood pressure signal occurring after a specified post-flush time window goes beyond one or more specified flush effectiveness threshold values. For example, the one or more specified flush effectiveness threshold values may include a systolic arterial blood pressure threshold value, a pulse arterial blood pressure threshold value, an arterial blood pressure waveform slope threshold value, the like, and/or combinations thereof.

In the illustrated example the arterial blood pressure management system 300 may further include the pressure sensor 112 sense the patient's arterial blood pressure and generate a signal and the automated valve 106 to automatically flush the catheter tubing 108. In some implementations, the pressure sensor input 114 may be implemented as a cable or wireless communication device coupled in communication with the pressure sensor 112. In some examples, the automated valve 106 may be an electronically-controllable fluid valve introduced in the flush tubing, which may replace a manual valve or be used in addition to a manual valve.

Additionally, or alternatively, the arterial blood pressure management system 300 may further include a therapeutic device 320, a medical management device 322, a database 324 (e.g., an EMR database), a user interface 326 (e.g., one or more user interfaces 326 may be associated with a user), the like (e.g., a patient monitor) and/or combinations thereof. For example, the therapeutic device 320, the medical management device 322, the database 324, and/or the user interface 326 may be in communication with one another via Internet based communicating, cloud based communication, wired communication, wireless communication, the like, and/or combinations thereof.

In an example, a patient monitor (not shown) may be configured to monitor a patient for vital signs and the like, and the patient monitor may communicate such measured patient data to the database 324. In some implementations, such a patient monitor may comprise a bedside-type monitor, a transport-type monitor, a central station-type monitor, the like, and/or combinations thereof.

In another example, the therapeutic device 320 may be configured to monitor the delivery of a particular therapy (e.g., a non-medication treatment) to a patient and may communicate such measured patient data to the database 324. In some implementations, the therapeutic device 320 may be coupled to the arterial blood pressure monitor 302. For example, the therapeutic device 320 may be a ventilator coupled to the arterial blood pressure monitor 302 as a closed-loop control system.

In a further example, the medical management device 322 may be configured to monitor medication delivery to a patient and may communicate such measured patient data to the database 324. In some implementations, the medical management device 322 may be coupled to the arterial blood pressure monitor 302. For example, the medical management device 322 may be a vasoactive medication delivery pump coupled to the arterial blood pressure monitor 302 as a closed-loop control system.

Additionally, or alternatively, in a still further example, the user interface 326 may be implemented via one or more formfactor devices (e.g., a smart phone, a tablet, a laptop, a workstation, and/or the like), an interface associated with the arterial blood pressure monitor 302, and/or an interface associated with a patient monitor. Additionally, or alternatively, a care provider receive patient data through an analog device, a non-networked patient monitor, a non-networked therapeutic device, a non-networked medical management device, the like, and/or combinations thereof.

In the illustrated implementation, the database 324 may include one or more types of patient data. For example, the database 324 may include patient data including laboratory result data, microbiology data, medication data, vital sign data, care order data, admission discharge and transfer data, and/or the like. As used herein, the term "database" refers to a collection of data and information organized in such a way as to allow the data and information to be stored, retrieved, updated, and/or manipulated. The term "database" as used herein may also refer to databases that may reside locally or that may be accessed from a remote location (e.g., via remote network servers).

As used herein, the term "patient data " refers to data or information for identifying an individual. Patient data may include measured patient data from an analog medical device, a sensor, a patient monitor, a therapeutic device, a medical management device, a medical imaging device, the like, and/or combinations thereof. Additionally, patient data may include a patient's name, age, weight, previous medical history, admission number, medical personnel in charge, date of admission, medical condition, a medical status, the like, and/or combinations thereof.

Additionally, or alternatively, in some implementations, the database 324 may include or be associated with a simulated database. In such an example, such a simulated database may generate estimated patient data. For example, the simulated database may utilize some measured patient data to generate some other estimated patient data. Such a simulated database may utilize digital twin technology to perform the estimation, for example. In such an example, such estimated patient data may be marked to indicate its estimated nature (rather than measured patient data). Additionally, or alternatively, a weight factor may be applied to the estimated patient data so that the estimated patient data may have a lower weight than corresponding measured patient data.

As used herein the term "closed-loop control system" refers to systems that rely on feedback to make automatic adjustments without ongoing input from a user. For example, once a user establishes parameters for a given system (e.g., establishing one or more set points, ranges of acceptable operation, thresholds, the like, and/or combinations thereof), such a closed-loop control system will utilize blood pressure feedback from the arterial blood pressure monitor 302 make automatic adjustments to a given therapy, drug delivery, the like, and/or combinations thereof.

In some implementations, the procedures described herein may be performed via one or more of a server, a smart hub, a smart cable, the arterial blood pressure monitor 302, the therapeutic device 320, the medical management device 322, the like, and/or combinations thereof.

As used herein the term "smart hub" refers to software, firmware, and/or hardware adapted to monitor and/or control a plurality of Internet-of-Things (IoT) devices. Additionally, or alternatively, such a smart hub will monitor and/or control interactions between individual devices among a plurality of Internet-of-Things (IoT) devices.

As used herein the term "smart cable" refers to a power cable and/or a communication cable that includes software, firmware, and/or hardware adapted to monitor and/or control one or more devices communicatively coupled to such a smart cable.

### ABP pulse detection and feature extraction

In operation, the ABP pulse detection and feature extraction techniques described herein may perform several tasks on analysing the ABP signal: pulse detection; pulse features extraction, signal quality assessment, the like, and/or combinations thereof.

For pulse detection, a slope-sum function based beat-pulse detection algorithm may be employed, which reliably detects the ABP pulse by its onset.

For pulse feature extraction, a group of features of the ABP pulses, such as inter-beat pulse interval, instantaneous ABP values and short-term averaged ABP values (e.g., including systolic, diastolic, mean, and pulse pressure) with signal quality control, instantaneous maximum ABP slopes and short-term averaged maximum ABP slopes (e.g., including the positive and negative) with signal quality control, may be extracted on a beat-to-beat basis and attached to each pulse.

For signal quality assessment, ABP signal quality is assessed for each detected pulse by analysing various ABP waveform features, and a signal quality index (SQI) value ranging from 0 to 1 (e.g., with 1 referring to the best quality, and 0 to the worst) may be determined and attached to the pulse.

### Detection of ABP damping event

In operation, the ABP damping detection techniques described herein may detect an ABP damping event by using the following features/signatures of the detected ABP waveform. For a given time window (e.g., 2 minutes):

1) The systolic ABP slowly decreases, from pulse to pulse;
2) The pulse ABP (i.e., the difference between the systolic and diastolic BP) slowly decreases, from pulse to pulse, to a significantly low level (e.g., 65%);
3) The mean ABP remains relatively unchanged, i.e., maintains at about the same level;
4) The diastolic ABP remains relatively unchanged, i.e., maintains at about the same level;
5) Most (e.g., 90%) of the ABP pulses have good signal quality (e.g., SQI > 0.9).

The output of the ABP damping detection techniques described herein is the flush necessity index (FNI) with binary value 0 or 1, with 1 for indicating a flush is required when the ABP waveform is dampened, and 0 for that the ABP waveform is not dampened.

A specific design and implementation of the ABP damping detection algorithm is as follows: Assuming, for a currently detected ABP pulse ($P_i$), there are N pulses detected in the time window ($T_w$) prior to $P_i$. The ABP features from those $N$ pulses are examined, and the following variables are defined and calculated. In accordance with one or more example embodiments, $T_w$ is 2 minutes (120s).

Percent of pulses with good signal quality in $T_w$:

$$P_{good\_SQI} = M / N \qquad\qquad (1),$$

where, N is the total number of pulse in the window ($T_w$), M is the number of pulses (in $T_w$) whose signal quality is good (i.e., the SQI value is above a predefined threshold, e.g., SQI > 0.7).

Percent of pulses with short-term averaged systolic BP (sBPa) declining in $T_w$:

$$P_{sBPa\_decline} = K \: / \: N \qquad (2),$$

where, $K$ is the number of pulses (in $T_w$) whose sBPa is lower than that of the previous pulse.

Percent of pulses with short-term averaged pulse BP (pBPa) declining in $T_w$:

$$P_{pBPa\_decline} = L \: / \: N; \qquad (3),$$

where, L is the number of pulses (in $T_w$) whose pBPa is lower than that of the previous pulse.

Difference of short-term averaged mean BP (mBPa) across $T_w$:

$$mBP_{i\_vs\_x} = mBPa(i) \: / \: mBPa(x) \qquad (4),$$

where, mBPa(i) is the mBPa value at the current pulse time (i), mBPa(x) is the mBPa value at time x, $x = i - T_w$

Difference of short-term averaged diastolic BP (dBPa) across $T_w$:

$$dBP_{i\_vs\_x} = dBPa(i) \: / \: dBPa(x); \qquad (5),$$

where, $dBPa(i)$ is the dBPa value at the current pulse time (i), $dBPa(x)$ is the dBPa value at time $x$, $x = i - T_w$.

Difference of short-term averaged pulse BP (pBPa) across $T_w$:

$$pBP_{i\_vs\_x} = pBPa(i) \: / \: pBPa(x); \qquad (6),$$

where, $pBPa(i)$ is the pBPa value at the current pulse time (i), $pBPa(x)$ is the pBPa value at time $x$, $x = i - T_w$.

The ABP flush necessity index (FNI) may be derived from the follow logic:

IF $\quad P_{good\_SQI} > thr1 \quad$ AND

$(P_{sBPa\_decline} > thr2 \quad$ OR $\quad P_{pBPa\_decline} > thr3) \quad$ AND

$(mBP_{i\_vs\_x} > thr4 \quad$ OR $\quad dBP_{i\_vs\_x} > thr5) \quad$ AND

$pBP_{i\_vs\_x} < thr6$

THEN $\quad$ FNI = 1;

ELSE $\quad$ FNI = 0; $\qquad (7),$

where, thr1, thr2, thr3, thr4, thr5, and thr6 are appropriate thresholds, which may be obtained empirically from experimental data. In this embodiment, thr1 is chosen as 0.9, thr2 as 0.6, thr3 as 0.6, thr4 as 0.85, thr5 as 0.9, and thr6 as 0.65.

The FNI may be calculated in a beat by beat manner.

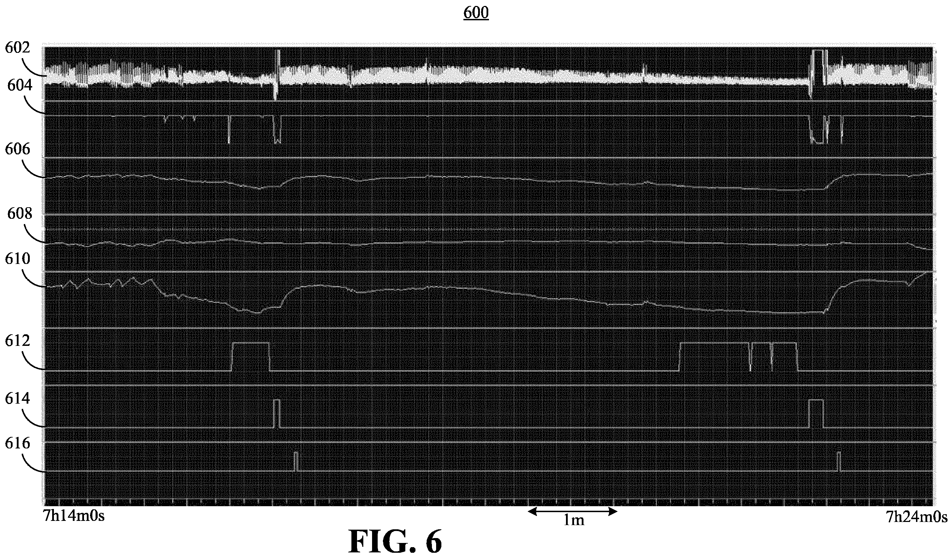

As will be described in greater detail below, FIG. 6 shows an example result of ABP damping detection. As illustrated, the ABP raw signal 602 panel is an episode of ABP raw signal recorded from an ICU patient; where there were two ABP damping events followed by manually made flushes by an ICU nurse (e.g., the manual flush events can be seen as a pulse where the pressure is saturated). The ABP SQI 604 panel illustrates the automated flush technology described herein generated ABP signal quality index (SQI) value. The Systolic ABPa 606, Diastolic ABPa 608, and Pulse ABPa 610, panels illustrate the automated flush technology described herein generated short-term averaged (e.g., with SQI control) systolic, diastolic, and pulse blood pressure values, respectively. The ABP damping (FNI) 612 panel is the *FNI* (Boolean) value generated by the ABP damping detection algorithm. Referring back to the ABP signal at the top, the ABP damping detection algorithm triggers before the manual flush event as desired, and the *FNI* signal remains true until the flush event.

Accordingly, the example of graph 600 illustrates that the automated flush technology described herein could take action before a skilled ICU nurse.

Alternatively, or additionally, in response to the determination that the arterial blood pressure signal damping event is occurring, the arterial blood pressure monitor 302 may be caused to alert the medical clinician or professional conducting the IABP monitoring by automatically generating one or more of an audio warning signal, a video warning signal, or a haptic warning signal. This alert of arterial blood pressure signal damping event may be recorded in the medical database system and may be used to suppress the related false positive blood pressure alarms.

Alternatively, or additionally, in response the determination that the arterial blood pressure signal damping event is occurring, the arterial blood pressure monitor 302 may be caused to automatically initiate a non-invasive arterial BP (NIABP) measurement of a subject S. The NIABP measurement may not be initiated in the event one or more recent NIABP measurements of the subject S is available and stored in one or more of the databases 324.

In accordance with one or more example embodiments, the arterial blood pressure management system 300 dynamically monitors the patient's ABP performance by considering one or more distinguishable patterns in an IABP waveform that are associated with damping. The flush effectiveness assessment (FEA) techniques described herein is applied to dynamically analyze the IABP waveform in real-time to automatically detect the one or more distinguishable patterns.

## Activation of automated flushing

In operation, when an ABP damping event is detected, a single pulse or a square-wave like flushing control signal may be issued to activate an auto-flushing control. The automated valve 106 is thus opened for a proper period of time (e.g., 2 second, or the like) and then shut down, so as to complete an auto-flushing operation control. The square wave-like flushing control signal allows multiple auto-flushing operations to happen when such multiple auto-flushing operations are needed.

For example, once damping is detected, the arterial blood pressure monitor 302 may send a first control signal to automatically initiate a fast-flushing sequence. The flush effectiveness assessment (FEA) techniques described herein continues to analyze the IABP waveform after the fast-flushing sequence and evaluates whether the detected, dampened IABP waveform is resolved (e.g., returned to normal). Should the dampened IABP waveform be resolved after the fast-flushing sequence, the IABP monitoring process continues.

In accordance with one or more example embodiments, the automatic fast-flushing sequence may be conducted via a flushing apparatus comprising automated valve 106 (e.g., a computer controlled electric fluid valve) in the flush tubing 104. Upon receiving an activation signal from the arterial blood pressure monitor 302, the arterial blood pressure management system 300 may make a timed (e.g., in terms of the time and duration) fast flush.

## Detection of ABP recovery (after the flush)

In operation, the arterial blood pressure monitor 302 may be configured to track the ABP waveform immediately after an automatic flushing sequence, and then determines whether the dampened ABP waveform is recovered, e.g., the flushing operation is effective. Recovery of the ABP waveform may be indicated by one or more distinguishable signatures, e.g., the systolic BP and pulse BP values are significantly enlarged, and the maximum ABP waveform slope is significantly increased.

In accordance with one or more embodiments, detection of ABP recovery by the arterial blood pressure monitor 302 after implementation of the automatic flushing sequence may be conducted by the arterial blood pressure monitor 302 initiating a flush effectiveness assessment (FEA) algorithm. The FEA algorithm may track certain ABP waveform features immediately following the automatic flushing sequence, and then compares them to those same features taken immediately before the automatic flushing sequence to determine if the ABP waveform is recovered.

A specific design and implementation of the FEA algorithm may be executed as follows. Assuming that the automatic flushing sequence start time is $F_{on}$. The following ABP features before the flush sequence are calculated from those ABP pulses in a period of $T_{b2}$ (e.g., 10s) which is located at $T_{b1}$ (e.g., 2s) before $F_{on}$:

sBPa_before_flush, which is the averaged systolic ABP in $T_{b2}$;
pBPa_before_flush, which is the averaged pulse ABP in $T_{b2}$;
mxSLPa_before_flush, which is the averaged maximum ABP waveform slope in $T_{b2}$, where only those ABP pulses in $T_{b2}$ with good signal quality (e.g., SQI > 0.9) are taken into account. In this embodiment, $T_{b2}$ is chosen as 10s; $T_{b1}$ as 2s, and SQI threshold as 0.9.

Assuming that the automatic flushing sequence end time is $F_{off}$. The following ABP features after the flush are calculated from the ABP pulses in the period of $T_{e2}$ (e.g., 10s) which is located at $T_{e1}$ (e.g., 3s) after $F_{off}$:

sBPa_after_flush, which is the averaged systolic ABP in $T_{e2}$;

pBPa_after_flush, which is the averaged pulse ABP in $T_{e2}$;

mxSLPa_after_flush, which is the averaged maximum ABP waveform slope in $T_{e2}$, where only those ABP pulses in $T_{e2}$ with good signal quality (e.g., SQI > 0.9) are taken into account. In accordance with one or more example embodiments, $T_{e2}$ is chosen as 10s; $T_{e1}$ as 3s, and SQI threshold as 0.9.

A flush effectiveness index (FEI) may be derived from the following logic:

$$\text{FEI} = 0;$$

$$\text{IF} \quad \text{pABPa\_after\_flush} / \text{pABPa\_before\_flush} \quad > r1 \quad \text{AND}$$

$$\text{sABPa\_after\_flush} / \text{sABPa\_before\_flush} \quad > r2 \quad \text{AND}$$

$$\text{mxSLPa\_after\_flush} / \text{mxSLPa\_before\_flush} \quad > r3 \quad \text{AND}$$

$$\text{THEN} \quad \text{FEI} = 1; \quad \text{(for 2s)}$$

$$\text{ELSE} \quad \text{FEI} = -1; \quad \text{(for 2s)} \quad \quad (8),$$

where, r1, r2, and r3 are appropriate (ratio) thresholds, which may be obtained empirically from experimental data. In accordance with one or more example embodiments, r1 is chosen as 1.5, r2 as 1.2, and r3 as 2.0.

The FEI value is initialized as 0. Should the automatic flushing sequence be effective (i.e., ABP signal recovered), the FEI receives a value of "1" (for 2s, and then returns to a value of 0, in order to visually illustrate the result). Should, on the other hand, the automatic flushing sequence not be successful, the FEI receives a value of "-1" (for 2s, and the returns to a value of 0, in order to visually illustrate the result). The judgement is made at the time $(T_{e1} + T_{e2})$ after $F_{off}$. This short delay is necessary due to the need of a reasonable period of time to reliably obtain the ABP features after the automatic flushing sequence. The skip windows $T_{b1}$ and $T_{e1}$, are introduced for excluding those ABP waveforms which are very close to (and thus, might be disturbed by) the automatic flushing sequence.

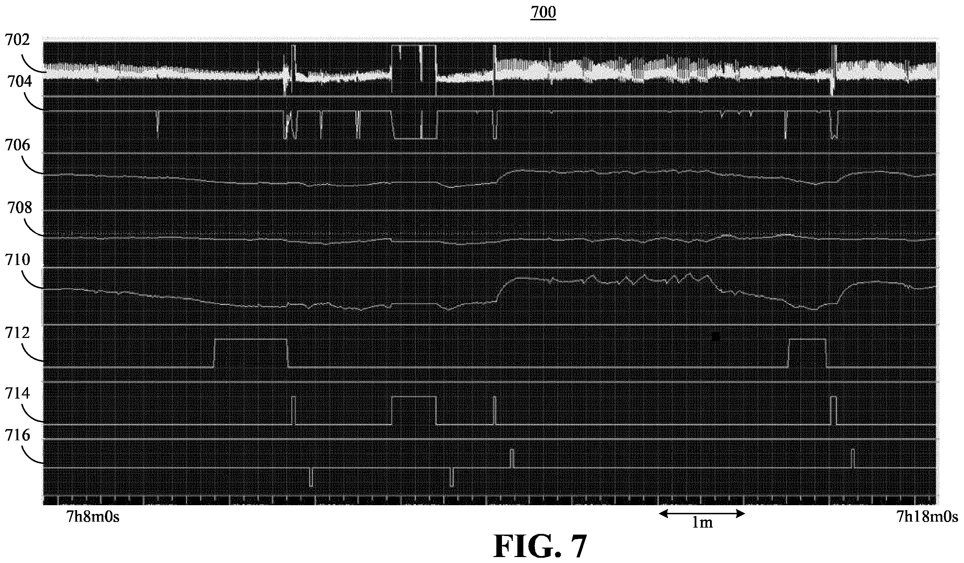

The FEI is calculated shortly after the end of each automatic flushing sequence. Additional details regarding the FEI results are described below with reference to FIGS. 6 and 7.

As will be described in greater detail below, in the illustrated example of FIG. 6, to illustrate the FEI result, a flushing signal was generated, as illustrated in a Flush operations 614 panel of FIG. 6, according to the manual flushing operations on the ABP record for a skilled ICU the medical clinician or professional. The flushing signal comprises a single pulse or a square-wave function, with its non-zero values corresponding to the manual flushing operations (e.g., as identified by the saturated ABP signal illustrated in a ABP raw signal 602 panel) for the medical clinician or professional. The proposed FEA algorithm executed by the arterial blood pressure monitor 302 takes in the onset time and offset time of each flushing operation and produced the FEI value at $(T_{e1} + T_{e2})$ after the flush offset time for this flush, as seen in a Flush effectiveness (FEI) 616 panel. The FEI value lasts for a duration of 2s and returns to zero for visually observing the result of the flush effectiveness assessment. As seen in the Flush effectiveness (FEI) 616 panel, both the flushes are correctly assessed as effective.

As will be described in greater detail below, in the illustrated example of FIG. 7, a plurality of flushes was performed for the IABP damping detection and flush effectiveness assessment. For the first IABP damping event, it is correctly detected by the arterial blood pressure monitor 302, as indicated by the FNI value in an ABP damping (FNI) 712 panel, also preceding a visual observation by the medical clinician or professional. The flush sequence comprises four manual flushes performed by the medical clinician or professional (as indicated in a Flush operations 714 panel). The first three flushes were corresponding to the first damping event. Of the first three flushes, the first two flushes were not effective, and the third flush was successful (e.g., effective). The proposed FEA algorithm executed by the arterial blood pressure monitor 302 correctly assessed the flushing effectiveness, by producing FEI with a value of -1 for the first two flushes and FEI with a value of 1 for the third flush (as indicated in Flush effectiveness (FEI) 716 panel). The fourth flush is corresponding to the second ABP damping event. The second IABP damping event is correctly detected by the arterial blood pressure monitor 302 before manual identification by the medical clinician or professional. The (fourth) manual flushing operation (at around (07:17:00) is correctly assessed as effective (as FEI has a value of 1).

In accordance with one or more example embodiments, the arterial blood pressure management system 300 may be configured to label the detected IABP data stream that is associated with the ABP damping event as "questionable," thereby suppressing the generation of an alarm based on a false positive physiological alarm pursuant to its logic.

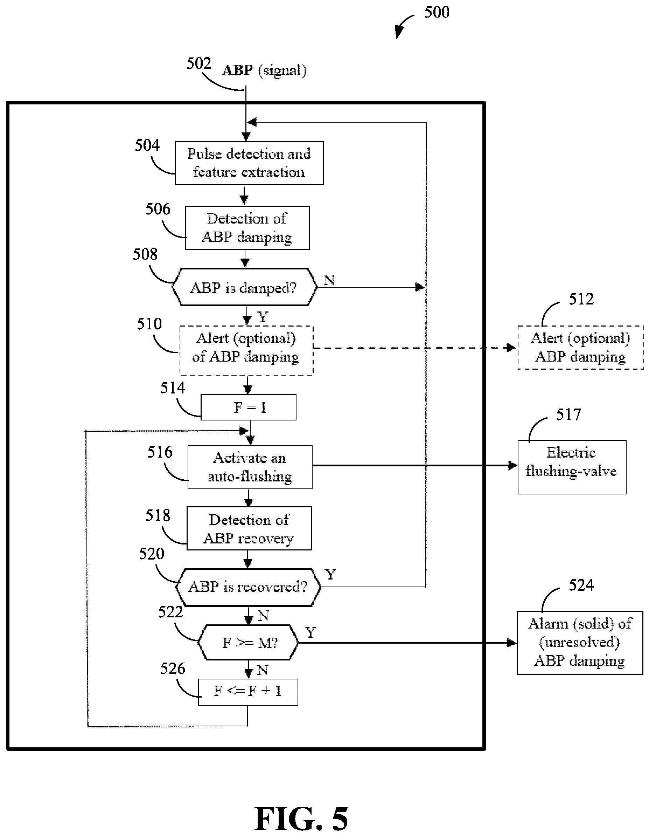

As will be discussed in greater detail below with reference to FIG. 5, should the ABP waveform be recovered after an automatic flushing sequence, the IABP monitoring continues. In the event the IABP is not recovered, a subsequent automatic flushing sequence may be performed. Should the number of the flushes in the sequence exceed a predefined or predetermined numeric value M (e.g., M = 3) and the IABP waveform is still not recovered, the arterial blood pressure management system 300 may cause the generation of an alarm (e.g., an audio warning signal, a video warning signal, a haptic warning signal, the like, and/or combinations thereof) to alert the medical clinician or professional by indicating that the catheter tubing 108 has an overdamping problem and needs manually intervention to resolve the damping issue.

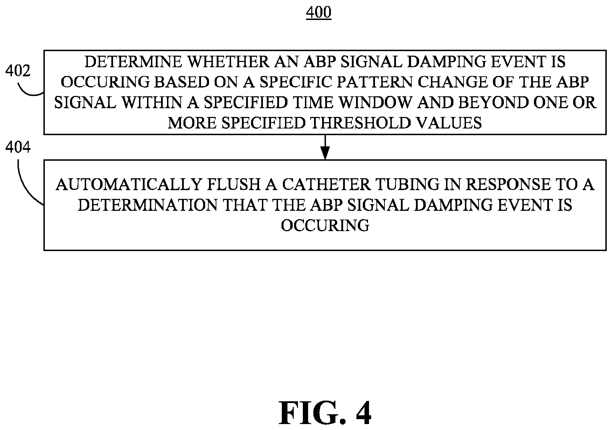

FIG. 4 shows an example method 400 for operating an arterial blood pressure management system according to an embodiment. The method 400 may generally be implemented in an arterial blood pressure management system, such as, for example, the arterial blood pressure management system 300 (FIG. 3), already discussed.

In an embodiment, the method 400 (as well as method 500 (FIG. 5) may be implemented in logic instructions (e.g., software), configurable logic, fixed-functionality hardware logic, etc., or any combination thereof. While certain portions of the EMR management system 300 (FIG. 3) operations are illustrated in method 400 (as well as method 500 (FIG. 5), other portions of the EMR management system 300 (FIG. 3) operations have been intentionally left out to simplify the explanation of the method.

Illustrated processing block 402 provides for determining whether an arterial blood pressure signal damping event is occurring. For example, such a determination may be based on a specific pattern change of the arterial blood pressure signal within a specified time window and beyond one or more specified threshold values.

Illustrated processing block 404 provides for automatically flushing a catheter tubing. For example, the catheter tubing may be automatically flushed in response to a determination that the arterial blood pressure signal damping event is occurring.

Additional and/or alternative operations for method 400 are described in greater detail below in the description of FIG. 5.

FIG. 5 is a flowchart of an example of another method 500 for operating an arterial blood pressure management system according to an embodiment. The method 500 may generally be implemented in an arterial blood pressure management system, such as, for example, the arterial blood pressure management system 300 (FIG. 3), already discussed.

Illustrated processing block 504 provides for pulse detection and feature extraction from signal 502. For example, pulse detection may be performed on an arterial blood pressure signal. Additionally, pulse features may be extracted from the arterial blood pressure signal.

In some implementations, a signal quality may be determined on a on a beat-to-beat basis associated with each pulse. For example, the pulse features may be extracted on a on a beat-to-beat basis associated with each pulse, where the pulse features may include one or more of: an inter-beat pulse interval, an instantaneous arterial blood pressure value, an averaged arterial blood pressure value, an instantaneous maximum arterial blood pressure slope, an averaged maximum arterial blood pressure slope, the like, and/or combinations thereof.

Illustrated processing block 506 provides for detection of arterial blood pressure damping. For example, such a determination of whether the arterial blood pressure signal damping event is occurring is further based on one or more of: determining whether there is a continuous decrease from beat-to-beat in a systolic arterial blood pressure based on a first one of the one or more specified threshold values; determining whether there is a continuous decrease from beat-to-beat in a difference between the systolic arterial blood pressure and a diastolic arterial blood pressure based on a second one of the one or more specified threshold values; determining whether a mean arterial blood pressure is consistent over time based on a third one of the one or more specified threshold values; determining whether the diastolic arterial blood pressure is consistent over time based on a fourth one of the one or more specified threshold values; determining whether a percentage of pulses with a signal quality beyond a signal quality threshold value within the specified time window is beyond a fifth one of the one or more specified threshold values, the like, and/or combinations thereof.

In some implementations, a determination that the arterial blood pressure signal damping event is occurring may be based on a first criterion and determining that the arterial blood pressure signal damping event is resolved is based on a second criterion that is different than the first criterion.

Illustrated processing block 508 provides for a decision block for whether arterial blood pressure is damped. In the situation where the arterial blood pressure signal damping event is not determined to be occurring, the method 500 returns to processing block 504. In the situation where the arterial blood pressure signal damping event is determined to be occurring, the method 500 proceeds to processing block 510.

Illustrated processing block 510 provides for triggering an optional alert of an arterial blood pressure signal damping event.

Additionally, a blood pressure limit alarm may be suppressed in response to the transfer of the damping event notification to the user interface

In the situation where the optional alert of arterial blood pressure signal damping event is triggered, the method 500 proceeds to processing block 512, otherwise the method 500 proceeds to processing block 514.

Illustrated processing block 512 provides for delivering the optional alert of arterial blood pressure signal damping event. For example, a damping event notification may be transferred to a user interface 326 associated with a care provider and/or other devices (e.g., the arterial blood pressure monitor 302, the therapeutic device 320, the medical management device 322, the like, and/or combinations thereof) in response to the determination that the arterial blood pressure signal damping event is occurring.

Illustrated processing block 514 provides for initializing a counter.

Illustrated processing block 516 provides for activating an auto flushing operation.

Illustrated processing block 517 provides for utilizing an electric flushing valve to perform the auto flushing operation.

Illustrated processing block 518 provides for detection of arterial blood pressure recovery. For example, a determination may be made as to whether the arterial blood pressure signal damping event is resolved in response to automatic flushing of the catheter tubing based on the arterial blood pressure signal. Such a determination that the arterial blood pressure signal damping event is resolved may include determining whether the arterial blood pressure signal occurring before a specified pre-flush time window compared to the arterial blood pressure signal occurring after a specified post-flush time window goes beyond one or more specified flush effectiveness threshold values.

In some implementations the one or more specified flush effectiveness threshold values may include a systolic arterial blood pressure threshold value, a difference between systolic and diastolic arterial blood pressure threshold value (e.g., a "pulse ABP"), a maximum arterial blood pressure waveform slope threshold value, the like, and/or combinations thereof

Illustrated processing block 520 provides for a decision block for whether arterial blood pressure recovery has occurred. In the situation where the arterial blood pressure recovery has occurred, the method 500 returns to processing block 504. In the situation where the arterial blood pressure recovery has not occurred, the method 500 proceeds to processing block 522.

Illustrated processing block 522 provides for a decision block for whether the counter has exceeded a preset maximum iteration value. In the situation where the counter has exceeded the preset maximum iteration value, the method 500 proceed to processing block 524. In the situation where the counter has not exceeded the preset maximum iteration value, the method 500 proceeds to processing block 526.

Illustrated processing block 524 provides for generating an alarm indicating that the arterial blood pressure recovery has not occurred. For example, such an alarm is generated after a limit of automated flushes has been performed and been unable to resolve arterial blood pressure damping. In some implementation, an unresolve flush notification may be transferred to the user interface 326 associated with a care provider and/or other devices (e.g., the arterial blood pressure monitor 302, the therapeutic device 320, the medical management device 322, the like, and/or combinations thereof) in response to determining that the arterial blood pressure signal damping event is unresolved.

Additionally, a blood pressure limit alarm may be suppressed in response to the transfer of the unresolve flush notification to the user interface.

Illustrated processing block 526 provides for incrementing the counter and returning to processing block 516.

In operation, the method 500 may be performed via one or more of a server, a smart hub, a smart cable, the arterial blood pressure monitor 302, the therapeutic device 320, the medical management device 322, the like, and/or combinations thereof.

It will be appreciated that some or all of the operations in method 500 above that have been described using a "pull" architecture (e.g., polling for new information followed by a corresponding response) may instead be implemented using a "push" architecture (e.g., sending such information when there is new information to report), and *vice versa*.

FIG. 6 shows an illustration of a graph 600 of an example detection of an arterial blood pressure damping event and a consequent assessment of the recovery for the damping event after a corresponding flush according to an embodiment. In the illustrated example, ABP raw signal 602, ABP SQI 604, Systolic ABPa 606, Diastolic ABPa 608, Pulse ABPa 610, ABP damping (FNI) 612, Flush operations 614 (e.g., manual flushes by a care provider), and Flush effectiveness (FEI) 616 are shown.

The graph 600 illustrates an example of ABP damping events due to clot formation along with corresponding flushes and return to normal pressure. The ABP damping events are correctly detected (e.g., as indicated by ABP damping (FNI) 612) by the techniques described herein (e.g., at earlier time than skilled nurse's observation). Both flushes are correctly assessed as effective by the techniques described herein (as indicated by Flush effectiveness (FEI) 616).

FIG. 7 shows another illustration of a graph 700 of an example detection of arterial blood pressure damping events and a consequent assessment of the recovery of the damping event after corresponding repeated flushes according to an embodiment. In the illustrated example, ABP raw signal 702, ABP SQI 704, Systolic ABPa 706, Diastolic ABPa 708, Pulse ABPa 710, ABP damping (FNI) 712, Flush operations 714 (e.g., manual flushes by a care provider), and Flush effectiveness (FEI) 716 are shown.

The graph 700 illustrates another example of ABP damping events due to clot formation along with corresponding flushes. The two ABP damping events are correctly detected (as indicated by ABP damping (FNI) 712) by the techniques described herein (e.g., at earlier time than nurse's observation). The effectiveness of the flushes (including both effective and ineffective flushes) is correctly assessed by the techniques described herein (as indicated by Flush effectiveness (FEI) 716).

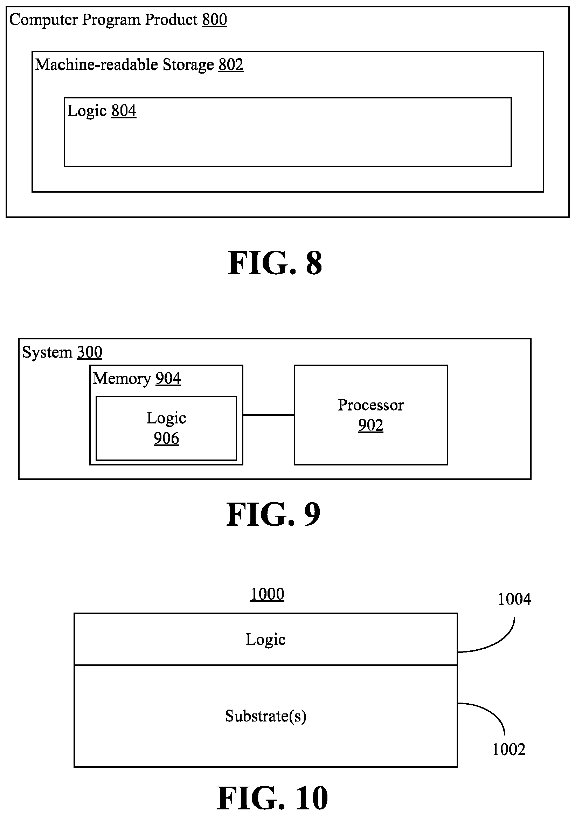

FIG. 8 illustrates a block diagram of an example computer program product 800. In some examples, as shown in FIG. 8, computer program product 800 includes a machine-readable storage 802 that may also include logic 804. In some implementations, the machine-readable storage 802 may be implemented as a non-transitory machine-readable storage. In some implementations the logic 804 may be implemented as machine-readable instructions, such as software, for example. In an embodiment, the logic 804, when executed, implements one or more aspects of the method 400 (FIG. 4), the method 500 (FIG. 5), and/or realize the arterial blood pressure management system 300 (FIG. 3), already discussed.

FIG. 9 shows an illustrative example of the arterial blood pressure management system 300. In the illustrated example, the arterial blood pressure management system 300 may include a processor 902 and a memory 904 communicatively coupled to the processor 902. The memory 904 may include logic 906 as a set of instructions. In some implementations the logic 906 may be implemented as software. In an embodiment, the logic 906, when executed by the processor 902, implements one or more aspects of the method 400 (FIG. 4), the method 500 (FIG. 5), and/or realize the arterial blood pressure management system 300 (FIG. 3), already discussed.

In some implementations, the processor 902 may include a general purpose controller, a special purpose controller, a storage controller, a storage manager, a memory controller, a micro-controller, a general purpose processor, a special purpose processor, a central processor unit (CPU), the like, and/or combinations thereof.

Further, implementations may include distributed processing, component/object distributed processing, parallel processing, the like, and/or combinations thereof. For example, virtual computer system processing may implement one or more of the methods or functionalities as described herein, and the processor 902 described herein may be used to support such virtual processing.

In some examples, the memory 904 is an example of a computer-readable storage medium. For example, memory 904 may be any memory which is accessible to the processor 902, including, but not limited to RAM memory, registers, and register files, the like, and/or combinations thereof. References to "computer memory" or "memory" should be interpreted as possibly being multiple memories. The memory may for instance be multiple memories within the same computer system. The memory may also be multiple memories distributed amongst multiple computer systems or computing devices.

FIG. 10 shows an illustrative semiconductor apparatus 1000 (e.g., chip and/or package). The illustrated apparatus 1000 includes one or more substrates 1002 (e.g., silicon, sapphire, or gallium arsenide) and logic 1004 (e.g., configurable logic and/or fixed-functionality hardware logic) coupled to the substrate(s) 1002. In an embodiment, the logic 1004 implements one or more aspects of the method 400 (FIG. 4), the method 500 (FIG. 5), and/or realize the arterial blood pressure management system 300 (FIG. 3), already discussed.

In some implementations, logic 1004 may include transistor array and/or other integrated circuit/IC components. For example, configurable logic and/or fixed-functionality hardware logic implementations of the logic 1004 may include configurable logic such as, for example, programmable logic arrays (PLAs), field programmable gate arrays (FPGAs), complex programmable logic devices (CPLDs), or fixed-functionality logic hardware using circuit technology such as, for example, application specific integrated circuit (ASIC), complementary metal oxide semiconductor (CMOS) or transistor-transistor logic (TTL) technology, the like, and/or combinations thereof.

All definitions, as defined and used herein, should be understood to control over dictionary definitions, and/or ordinary meanings of the defined terms.

The herein described subject matter sometimes illustrates different components contained within, or connected with, different other components. It is to be understood that such depicted architectures are merely exemplary, and that in fact many other architectures can be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. The term "coupled" may be used herein to refer to any type of relationship, direct or indirect, between the components in question, and may apply to electrical, mechanical, fluid, optical, electromagnetic, electromechanical, or other connections. Likewise, any two components so associated can also be viewed as being "operably connected", or "operably coupled", to each other to achieve the desired functionality, and any two components capable of being so associated can also be viewed as being "operably couplable", to each other to achieve the desired functionality. Specific examples of operably couplable include but are not limited to physically mateable and/or physically interacting components.

In the claims, as well as in the specification above, the terms "first", "second", etc. may be used herein only to facilitate discussion, and carry no particular temporal or chronological significance unless otherwise indicated.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

The indefinite articles "a" and "an," as used herein in the specification and in the claims, unless clearly indicated to the contrary, should be understood to mean "at least one."

As used herein, the term "or" or "and/or" is inclusive and not exclusive, unless expressly indicated otherwise or indicated otherwise by context. Therefore, herein, "A or B" means "A, B, or both," unless expressly indicated otherwise or indicated otherwise by context. Moreover, "and" is both joint and several, unless expressly indicated otherwise or indicated otherwise by context. Therefore, herein, "A and B" means "A and B, jointly or severally," unless expressly indicated otherwise or indicated otherwise by context.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used in this application and in the claims, a list of items joined by the term "one or more of" may mean any combination of the listed terms. For example, the phrases "one or more of A, B or C" may mean A; B; C; A and B; A and C; B and C; or A, B and C.

As is described above in greater detail, one or more processor, other unit, the like, and/or combinations thereof may fulfill the functions of several items recited in the claims.

As is described above in greater detail, a computer program may be stored/distributed on a suitable medium, such as an optical storage medium or a solid-state medium supplied together with or as part of other hardware, but may also be distributed in other forms, such as via the Internet or other wired or wireless telecommunication systems.

It should also be understood that, unless clearly indicated to the contrary, in any methods discussed herein that include more than one step or act, the order of the steps or acts of the method is not necessarily limited to the order in which the steps or acts of the method are recited. Further, such methods may include additional or alternative steps or acts.

As used in the claims, the mere fact that certain measures are recited in mutually different dependent claims does not indicate that a combination of these measured cannot be used to advantage.

It is also noted that the claims may include reference signs/numerals in accordance with PCT Rule 6.2(b). However, the present claims should not be considered to be limited to the exemplary embodiments corresponding to the reference signs/numerals.

Those skilled in the art will appreciate from the foregoing description that the broad techniques of the embodiments of the present invention can be implemented in a variety of forms. Therefore, while the embodiments of this invention have been described in connection with particular examples thereof, the invention is not limited thereto but it is defined by the scope of the appended claims.

## Claims

1. An arterial blood pressure management system (300), comprising:

   a pressure sensor receptor (114) configured to receive an arterial blood pressure signal detected by a pressure sensor (112); and
   an arterial blood pressure monitor (302) communicatively coupled to the pressure sensor receptor, the arterial blood pressure monitor being configured to:

   determine whether an arterial blood pressure signal damping event is occurring based on the arterial blood pressure signal,
   transmit an instruction to an electronically controllable valve (106) to control the valve to automatically flush a catheter tubing (108) in response to a determination that the arterial blood pressure signal damping event is occurring,
   determine whether the arterial blood pressure signal damping event is resolved in response to the automatic flush of the catheter tubing based on the arterial blood pressure signal, wherein the determination that the arterial blood pressure signal damping event is occurring is based on a first criterion and a determination that the arterial blood pressure signal damping event is resolved is based on a second criterion that is different than the first criterion,
   determine, with regard to the first criterion, whether the arterial blood pressure signal damping event is occurring based on a pattern change of the arterial blood pressure signal within a specified time window and beyond one or more specified threshold values, and
   determine, with regard to the second criterion, whether the arterial blood pressure signal occurring before a specified pre-flush time window compared to the arterial blood pressure signal occurring after a specified post-flush time window goes beyond one or more specified flush effectiveness threshold values, where in the one or more specified flush effectiveness threshold values comprise a systolic arterial blood pressure threshold value, a pulse arterial blood pressure threshold value, and an arterial blood pressure waveform slope threshold value.

2. The arterial blood pressure management system of claim 1, further comprising the pressure sensor (112) configured to sense the patient's arterial blood pressure signal and the electronically controllable valve (106) configured to automatically flush the catheter tubing.

3. A method for operating an arterial blood pressure monitor (302), the method comprising:

determining whether an arterial blood pressure signal damping event is occurring based on an arterial blood pressure signal detected by a pressure sensor (112),
transmitting an instruction to an electronically controllable valve (106) to control the valve to automatically flush a catheter tubing (108) in response to a determination that the arterial blood pressure signal damping event is occurring,
determining whether the arterial blood pressure signal damping event is resolved in response to the automatic flush of the catheter tubing based on the arterial blood pressure signal, wherein the determination that the arterial blood pressure signal damping event is occurring is based on a first criterion and a determination that the arterial blood pressure signal damping event is resolved is based on a second criterion that is different than the first criterion,
determining, with regard to the first criterion, whether the arterial blood pressure signal damping event is occurring based on a pattern change of the arterial blood pressure signal within a specified time window and beyond one or more specified threshold values, and
determining, with regard to the second criterion, whether the arterial blood pressure signal occurring before a specified pre-flush time window compared to the arterial blood pressure signal occurring after a specified post-flush time window goes beyond one or more specified flush effectiveness threshold values, where in the one or more specified flush effectiveness threshold values comprise a systolic arterial blood pressure threshold value, a pulse arterial blood pressure threshold value, and an arterial blood pressure waveform slope threshold value.

4. The method of claim 3, further comprising:

performing pulse detection to generate the arterial blood pressure signal;
extracting pulse features from the arterial blood pressure signal; and
determining a signal quality on a on a beat-to-beat basis associated with each pulse.

5. The method of claim 4, wherein the pulse features are extracted on a on a beat-to-beat basis associated with each pulse, and wherein the pulse features include one or more of: an inter-beat pulse interval, an instantaneous arterial blood pressure value, an averaged arterial blood pressure value, an instantaneous maximum arterial blood pressure slope, and an averaged maximum arterial blood pressure slope.

6. The method of claim 3, wherein determination of whether the arterial blood pressure signal damping event is occurring is further based on one or more of:

determining whether there is a continuous decrease from beat-to-beat in a systolic arterial blood pressure based on a first one of the one or more specified threshold values;
determining whether there is a continuous decrease from beat-to-beat in a difference between the systolic arterial blood pressure and a diastolic arterial blood pressure based on a second one of the one or more specified threshold values;
determining whether a mean arterial blood pressure is consistent over time based on a third one of the one or more specified threshold values;
determining whether the diastolic arterial blood pressure is consistent over time based on a fourth one of the one or more specified threshold values; and
determining whether a percentage of pulses with a signal quality beyond a signal quality threshold value within the specified time window is beyond a fifth one of the one or more specified threshold values.

7. The method of claim 3, further comprising:
transferring a damping event notification to a user interface associated with a care provider in response to the determination that the arterial blood pressure signal damping event is occurring, wherein a blood pressure limit alarm is suppressed in response to the transfer of the damping event notification to the user interface.

8. The method of claim 3, further comprising:
transferring an unresolve flush notification to a user interface associated with a care provider in response to determining that the arterial blood pressure signal damping event is unresolved, wherein a blood pressure limit alarm is suppressed in response to the transfer of the unresolve flush notification to the user interface.

9. The method of claim 3, wherein the method is performed via one or more of a server, a smart hub, a smart cable, an arterial blood pressure monitor, a therapeutic device, and a medical management device.

10. A machine-readable storage including machine-readable instructions, which when executed, implement the method according to any one of claims 3-8, or realize the system according to any one of claims 1-2.

11. An apparatus, comprising:
means for performing the method according to any one of claims 3-8.

**Patentansprüche**

1. Managementsystem (300) für arteriellen Blutdruck, umfassend:

einen Drucksensorempfänger (114), der dazu konfiguriert ist, ein von einem Drucksensor (112) erfasstes Signal für arteriellen Blutdruck zu empfangen; und
ein Messgerät (302) für arteriellen Blutdruck, das kommunikativ mit dem Drucksensorempfänger gekoppelt ist, wobei das Messgerät für arteriellen Blutdruck konfiguriert ist zum:

Bestimmen, basierend auf dem Signal für arteriellen Blutdruck, ob ein Signaldämpfungsereignis für arteriellen Blutdruck auftritt,
Übertragen einer Anweisung an ein elektronisch steuerbares Ventil (106), um das Ventil so zu steuern, dass es einen Katheterschlauch (108) als Reaktion darauf automatisch spült, dass bestimmt wird, dass das Signaldämpfungsereignis für arteriellen Blutdruck auftritt,
Bestimmen, basierend auf dem Signal für arteriellen Blutdruck, ob das Signaldämpfungsereignis für arteriellen Blutdruck als Reaktion auf die automatische Spülung des Katheterschlauchs behoben ist, wobei die Bestimmung, dass das Signaldämpfungsereignis für arteriellen Blutdruck auftritt, auf einem ersten Kriterium basiert und eine Bestimmung, dass das Signaldämpfungsereignis für arteriellen Blutdruck behoben ist, auf einem zweiten Kriterium basiert, das sich von dem ersten Kriterium unterscheidet,
Bestimmen, im Hinblick auf das erste Kriterium, ob das Signaldämpfungsereignis für arteriellen Blutdruck auftritt, basierend auf einer Musteränderung des Signals für arteriellen Blutdruck innerhalb eines festgelegten Zeitfensters und über einen oder mehrere festgelegte Schwellenwerte hinaus, und
Bestimmen, im Hinblick auf das zweite Kriterium, ob das vor einem festgelegten Zeitfenster vor der Spülung auftretende Signal für arteriellen Blutdruck im Vergleich zu dem nach einem festgelegten Zeitfenster nach der Spülung auftretenden Signal für arteriellen Blutdruck einen oder mehrere festgelegte Schwellenwerte für Spülwirksamkeit überschreitet, wobei der eine oder die mehreren festgelegten Schwellenwerte für Spülwirksamkeit einen Schwellenwert für den systolischen arteriellen Blutdruck, einen Schwellenwert für den arteriellen Pulsblutdruck und einen Schwellenwert für die Steigung der Wellenform des arteriellen Blutdrucks umfassen.

2. Managementsystem für arteriellen Blutdruck nach Anspruch 1, das weiter den Drucksensor (112) umfasst, der zum Erfassen des Signals für arteriellen Blutdruck des Patienten konfiguriert ist, und das elektronisch steuerbare Ventil (106), das zum automatischen Spülen des Katheterschlauchs konfiguriert ist.

3. Verfahren zum Betreiben eines Messgeräts (302) für arteriellen Blutdruck, wobei das Verfahren umfasst:

Bestimmen, basierend auf einem von einem Drucksensor (112) erfassten Signal für arteriellen Blutdruck, ob ein Signaldämpfungsereignis für arteriellen Blutdruck auftritt,
Übertragen einer Anweisung an ein elektronisch steuerbares Ventil (106), um das Ventil so zu steuern, dass es einen Katheterschlauch (108) als Reaktion darauf automatisch spült, dass bestimmt wird, dass das Signaldämpfungsereignis für arteriellen Blutdruck auftritt,

Bestimmen, basierend auf dem Signal für arteriellen Blutdruck, ob das Signaldämpfungsereignis für arteriellen Blutdruck als Reaktion auf die automatische Spülung des Katheterschlauchs behoben ist, wobei die Bestimmung, dass das Signaldämpfungsereignis für arteriellen Blutdruck auftritt, auf einem ersten Kriterium basiert und eine Bestimmung, dass das Signaldämpfungsereignis für arteriellen Blutdruck behoben ist, auf einem zweiten Kriterium basiert, das sich von dem ersten Kriterium unterscheidet,

Bestimmen, im Hinblick auf das erste Kriterium, ob das Signaldämpfungsereignis für arteriellen Blutdruck auftritt, basierend auf einer Musteränderung des Signals für arteriellen Blutdruck innerhalb eines festgelegten Zeitfensters und über einen oder mehrere festgelegte Schwellenwerte hinaus, und

Bestimmen, im Hinblick auf das zweite Kriterium, ob das vor einem festgelegten Zeitfenster vor der Spülung auftretende Signal für arteriellen Blutdruck im Vergleich zu dem nach einem festgelegten Zeitfenster nach der Spülung auftretenden Signal für arteriellen Blutdruck einen oder mehrere festgelegte Schwellenwerte für Spülwirksamkeit überschreitet, wobei der eine oder die mehreren festgelegten Schwellenwerte für Spülwirksamkeit einen Schwellenwert für den systolischen arteriellen Blutdruck, einen Schwellenwert für den arteriellen Pulsblutdruck und einen Schwellenwert für die Steigung der Wellenform des arteriellen Blutdrucks umfassen.

4. Verfahren nach Anspruch 3, weiter umfassend:

Durchführen einer Pulserkennung zum Erzeugen des Signals für arteriellen Blutdruck;
Extrahieren von Pulsmerkmalen aus dem Signal für arteriellen Blutdruck; und
Bestimmen einer Signalqualität auf einer Herzschlag-zu-Herzschlag-Basis, die jedem Impuls zugeordnet ist.

5. Verfahren nach Anspruch 4, wobei die Pulsmerkmale auf einer jedem Puls zugeordneten Herzschlag-zu-Herzschlag-Basis extrahiert werden und wobei die Pulsmerkmale eines oder mehrere einschließen von: einem Pulsintervall zwischen Herzschlägen, einem momentanen Wert des arteriellen Blutdrucks, einem gemittelten Wert des arteriellen Blutdrucks, einer momentanen maximalen Steigung des arteriellen Blutdrucks und einer gemittelten maximalen Steigung des arteriellen Blutdrucks.

6. Verfahren nach Anspruch 3, wobei Bestimmung, ob das Signaldämpfungsereignis für arteriellen Blutdruck auftritt, weiter auf einem oder mehreren der Folgenden basiert:

Bestimmen, basierend auf einem ersten des einen oder der mehreren festgelegten Schwellenwerte, ob eine kontinuierliche Abnahme des systolischen arteriellen Blutdrucks von Herzschlag zu Herzschlag vorliegt;
Bestimmen, basierend auf einem zweiten des einen oder der mehreren festgelegten Schwellenwerte, ob eine kontinuierliche Abnahme einer Differenz zwischen dem systolischen arteriellen Blutdruck und einem diastolischen arteriellen Blutdruck von Herzschlag zu Herzschlag vorliegt;
Bestimmen, basierend auf einem dritten des einen oder der mehreren festgelegten Schwellenwerte, ob ein mittlerer arterieller Blutdruck im Zeitverlauf konstant ist;
Bestimmen, basierend auf einem vierten des einen oder der mehreren festgelegten Schwellenwerte, ob der diastolische arterielle Blutdruck im Zeitverlauf konstant ist; und
Bestimmen, ob ein Prozentsatz von Impulsen mit einer Signalqualität über einem Schwellenwert für die Signalqualität innerhalb des festgelegten Zeitfensters über einem fünften des einen oder der mehreren festgelegten Schwellenwerte liegt.

7. Verfahren nach Anspruch 3, weiter umfassend:
Übertragen einer Dämpfungsereignisbenachrichtigung an eine Benutzerschnittstelle, die einem Leistungserbringer zugeordnet ist, als Reaktion auf die Bestimmung, dass das Signaldämpfungsereignis für arteriellen Blutdruck auftritt, wobei als Reaktion auf die Übertragung der Dämpfungsereignisbenachrichtigung an die Benutzerschnittstelle ein Blutdruckgrenzwertalarm unterdrückt wird.

8. Verfahren nach Anspruch 3, weiter umfassend:
Übertragen einer Benachrichtigung über eine nicht behobene Spülung an eine Benutzerschnittstelle, die einem Leistungserbringer zugeordnet ist, als Reaktion auf Bestimmen, dass das Signaldämpfungsereignis für arteriellen Blutdruck nicht behoben ist, wobei als Reaktion auf die Übertragung der Benachrichtigung über eine nicht behobene Spülung an die Benutzerschnittstelle ein Blutdruckgrenzwertalarm unterdrückt wird.

9. Verfahren nach Anspruch 3, wobei das Verfahren über einen oder mehrere von einem Server, einem Smart Hub, einem Smart Cable, einem Messgerät für arteriellen Blutdruck, einer Therapievorrichtung und einer medizinischen Managementvorrichtung durchgeführt wird.

10. Maschinenlesbarer Speicher, der maschinenlesbare Anweisungen einschließt, die bei Ausführung das Verfahren nach einem der Ansprüche 3-8 implementieren oder das System nach einem der Ansprüche 1-2 umsetzen.

11. Einrichtung, umfassend:
    Mittel zum Durchführen des Verfahrens nach einem der Ansprüche 3-8.

**Revendications**

1. Système de gestion de pression artérielle (300), comprenant :

   un récepteur de capteur de pression (114) configuré pour recevoir un signal de pression artérielle détecté par un capteur de pression (112) ; et
   un moniteur de pression artérielle (302) couplé en communication au récepteur de capteur de pression, le moniteur de pression artérielle étant configuré pour :

   déterminer si un événement d'amortissement de signal de pression artérielle se produit sur la base du signal de pression artérielle,
   transmettre une instruction à une vanne à commande électronique (106) pour commander la vanne pour rincer automatiquement un tube de cathéter (108) à la suite d'une détermination que l'événement d'amortissement de signal de pression artérielle se produit,
   déterminer si l'événement d'amortissement de signal de pression artérielle est résolu à la suite du rinçage automatique du tube de cathéter sur la base du signal de pression artérielle, dans lequel la détermination que l'événement d'amortissement de signal de pression artérielle se produit, est basée sur un premier critère et une détermination que l'événement d'amortissement de signal de pression artérielle est résolu, est basée sur un second critère qui est différent du premier critère,
   déterminer, concernant le premier critère, si l'événement d'amortissement de signal de pression artérielle se produit sur la base d'un changement de modèle du signal de pression artérielle dans une fenêtre temporelle spécifiée et au-delà d'une ou de plusieurs valeurs seuils spécifiées, et
   déterminer, concernant le second critère, si le signal de pression artérielle se produisant avant une fenêtre temporelle de pré-rinçage spécifiée par rapport au signal de pression artérielle se produisant après une fenêtre temporelle de post-rinçage spécifiée, dépasse une ou plusieurs valeurs seuils d'efficacité de rinçage spécifiées, dans lequel les une ou plusieurs valeurs seuils d'efficacité de rinçage spécifiées comprennent une valeur seuil de pression artérielle systolique, une valeur seuil de pression artérielle pulsée et une valeur seuil de pente de forme d'onde de pression artérielle.

2. Système de gestion de pression artérielle selon la revendication 1, comprenant en outre le capteur de pression (112) configuré pour détecter le signal de pression artérielle du patient et la vanne à commande électronique (106) configurée pour rincer automatiquement le tube de cathéter.

3. Procédé de fonctionnement d'un moniteur de pression de pression artérielle (302), le procédé comprenant :

   la détermination pour savoir si un événement d'amortissement de signal de pression artérielle se produit sur la base d'un signal de pression artérielle détecté par un capteur de pression (112),
   la transmission d'une instruction à une vanne à commande électronique (106) pour commander la vanne pour rincer automatiquement un tube de cathéter (108) à la suite d'une détermination que l'événement d'amortissement de signal de pression artérielle se produit,
   la détermination pour savoir si l'événement d'amortissement de signal de pression artérielle est résolu à la suite du rinçage automatique du tube de cathéter sur la base du signal de pression artérielle, dans lequel la détermination que l'événement d'amortissement de signal de pression artérielle se produit, est basée sur un premier critère et une détermination que l'événement d'amortissement de signal de pression artérielle est résolu, est basée sur un second critère qui est différent du premier critère,
   la détermination, concernant le premier critère, pour savoir si l'événement d'amortissement de signal de pression artérielle se produit sur la base d'un changement de modèle du signal de pression artérielle dans une fenêtre temporelle spécifiée et au-delà d'une ou de plusieurs valeurs seuils spécifiées, et
   la détermination, concernant le second critère, pour savoir si le signal de pression artérielle se produisant avant une fenêtre temporelle de pré-rinçage spécifiée par rapport au signal de pression artérielle se produisant après une fenêtre temporelle de post-rinçage spécifiée dépasse une ou plusieurs valeurs seuils d'efficacité de rinçage spécifiées, dans lequel les une ou plusieurs valeurs seuils d'efficacité de rinçage spécifiées comprennent une valeur seuil de pression artérielle systolique, une valeur seuil de pression artérielle pulsée et une valeur seuil de pente de forme d'onde de pression artérielle.

4. Procédé selon la revendication 3, comprenant en outre :

la réalisation d'une détection de pulsation pour générer le signal de pression artérielle ;
l'extraction de caractéristiques de pulsation à partir du signal de pression artérielle ; et
la détermination d'une qualité de signal sur une base de battement par battement associée à chaque pulsation.

5. Procédé selon la revendication 4, dans lequel les caractéristiques de pulsation sont extraites sur une base de battement par battement associée à chaque pulsation, et dans lequel les caractéristiques de pulsation incluent un ou plusieurs : d'un intervalle de pulsation inter-battement, d'une valeur de pression artérielle instantanée, d'une valeur de pression artérielle moyenne, d'une pente de pression artérielle maximale instantanée et d'une pente de pression artérielle maximale moyenne.

6. Procédé selon la revendication 3, dans lequel la détermination pour savoir si l'événement d'amortissement de signal de pression artérielle se produit ou non, est en outre basée sur une ou plusieurs :

de la détermination pour savoir s'il y a, ou non, une diminution continue d'un battement à battement de la pression artérielle systolique sur la base d'une première valeur seuil spécifiée des une ou plusieurs valeurs seuils spécifiées ;
de la détermination pour savoir s'il y a, ou non, une diminution continue d'un battement à battement d'une différence entre la pression artérielle systolique et la pression artérielle diastolique sur la base d'une deuxième valeur seuil spécifiée des une ou plusieurs valeurs seuils spécifiées ;
de la détermination pour savoir si une pression artérielle moyenne est, ou non, constante dans le temps sur la base d'une troisième valeur seuil spécifiée des une ou plusieurs valeurs seuils spécifiées ;
de la détermination pour savoir si la pression artérielle diastolique est, ou non, constante dans le temps sur la base d'une quatrième valeur seuil spécifiée des une ou plusieurs valeurs seuils spécifiées ; et
de la détermination pour savoir si un pourcentage de pulsations avec une qualité de signal au-delà d'une valeur seuil de qualité de signal dans la fenêtre temporelle spécifiée est au-delà d'une cinquième valeur seuil spécifiée des une ou plusieurs valeurs seuils spécifiées.

7. Procédé selon la revendication 3, comprenant en outre :
le transfert d'une notification d'événement d'amortissement à une interface utilisateur associée à un prestataire de soins à la suite de la détermination que l'événement d'amortissement de signal de pression artérielle se produit, dans lequel une alarme de limite de pression artérielle est supprimée à la suite du transfert de la notification d'événement d'amortissement à l'interface utilisateur.

8. Procédé selon la revendication 3, comprenant en outre :
le transfert d'une notification de rinçage non résolu à une interface utilisateur associée à un prestataire de soins à la suite de la détermination que l'événement d'amortissement de signal de pression artérielle est non résolu, dans lequel une alarme de limite de pression artérielle est supprimée à la suite du transfert de la notification de rinçage non résolu à l'interface utilisateur.

9. Procédé selon la revendication 3, dans lequel le procédé est réalisé au moyen d'un ou de plusieurs d'un serveur, d'un concentrateur intelligent, d'un câble intelligent, d'un moniteur de pression artérielle, d'un dispositif thérapeutique et d'un dispositif de gestion médicale.

10. Stockage lisible par machine incluant des instructions lisibles par machine qui, lorsqu'elles sont exécutées, mettent en œuvre le procédé selon l'une quelconque des revendications 3-8, ou réalisent le système selon l'une quelconque des revendications 1-2.

11. Appareil, comprenant :
des moyens pour réaliser le procédé selon l'une quelconque des revendications 3-8.

**FIG. 1**

FIG. 2

**FIG. 3**

400

| |
|---|
| **402** DETERMINE WHETHER AN ABP SIGNAL DAMPING EVENT IS OCCURING BASED ON A SPECIFIC PATTERN CHANGE OF THE ABP SIGNAL WITHIN A SPECIFIED TIME WINDOW AND BEYOND ONE OR MORE SPECIFIED THRESHOLD VALUES |
| **404** AUTOMATICALLY FLUSH A CATHETER TUBING IN RESPONSE TO A DETERMINATION THAT THE ABP SIGNAL DAMPING EVENT IS OCCURING |

# FIG. 4

FIG. 5

**FIG. 6**

**FIG. 7**

Computer Program Product 800

Machine-readable Storage 802

Logic 804

# FIG. 8

System 300

Memory 904

Logic
906

Processor
902

# FIG. 9

1000

1004

Logic

Substrate(s)

1002

# FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015126880 A1

- US 2010094113 A1